# EUROPÄISCHE PATENTSCHRIFT

(11) **EP 1 476 582 B1**
(45) Veröffentlichungstag und Bekanntmachung des Hinweises auf die Patenterteilung: **17.09.2008**
(21) Anmeldenummer: 03742490.0
(22) Anmeldetag: 13.02.2003
(51) Int. Cl.: C12R 1/42, C12R 1/19, C07K 14/195, A61K 39/02

(54) **NUKLEOTIDSEQUENZ CODIEREND FÜR EIN TOLC, DAS EINE HETEROLOGE AMINOSÄURESEQUENZ ENTHÄLT**
NUCLEOTIDE SEQUENCE CODING FOR A TOLC CONTAINING A HETEROLOGOUS AMINO ACID SEQUENCE
SEQUENCE NUCLEOTIDIQUE CODANT POUR UNE TOLC CONTENANT UNE SEQUENCE D'ACIDE AMINE HETEROLOGUE

(30) Priorität: 20.02.2002 DE 10208175
(43) Veröffentlichungstag der Anmeldung: 17.11.2004
(73) Patentinhaber: AEterna Zentaris GmbH, 60314 Frankfurt (DE)
(72) Erfinder: GOEBEL, Werner, 97218 Gerbrunn (DE); GENTSCHEV, Ivaylo, 97270 Kist (DE); SPRENG, Simone, CH-3007 Bern (CH)
(86) Internationale Anmeldenummer: PCT/DE2003/000469
(87) Internationale Veröffentlichungsnummer: WO 2003/070987

(56) Entgegenhaltungen:
- KORONAKIS V ET AL: "Crystal structure of the bacterial membrane protein TolC central to multidrug efflux and protein export." NATURE, Bd. 405, Nr. 6789, 22. Juni 2000 (2000-06-22), Seiten 914-919, XP002246208 ISSN: 0028-0836 in der Anmeldung erwähnt
- HORMAECHE C E ET AL: "SALMONELLA VACCINES: MECHANISMS OF IMMUNITY AND THEIR USE AS CARRIERS OF RECOMBINANT ANTIGENS" MOLECULAR AND CLINICAL ASPECTS OF BACTERIAL VACCINE DEVELOPMENT, 1995, Seiten 119-153, XP002054901
- GENTSCHEV I ET AL: "Development of antigen-delivery systems, based on the Escherichia coli hemolysin secretion pathway" GENE, Bd. 179, Nr. 1, 7. November 1996 (1996-11-07), Seiten 133-140, XP002078662 ISSN: 0378-1119 in der Anmeldung erwähnt
- GENTSCHEV I ET AL: "Delivery of protein antigens and DNA by virulence-attenuated strains of Salmonella typhimurium and Listeria monocytogenes" JOURNAL OF BIOTECHNOLOGY, Bd. 83, Nr. 1-2, 29. September 2000 (2000-09-29), Seiten 19-26, XP004212147 ISSN: 0168-1656

## Beschreibung

### Gebiet der Erfindung.

Die Erfindung betrifft eine Nukleotidsequenz codierend für ein TolC, ein Plasmid enthaltend eine solche Nukleotidsequenz, ein Protein oder ein Peptid codiert durch eine solche Nukleotidsequenz, ein Bakterium enthaltend eine solche Nukleotidsequenz sowie verschiedene Verwendungen solcher Bakterien.

### Hintergrund der Erfindung und Stand der Technik.

In ihrer Virulenz attenuierte, sich intrazellulär ansiedelnde Bakterien können als Lebendvakzinen eine langanhaltende Immunität induzieren. Bislang wurden besonders Salmonella Typhi TYla (Levine et al., Lancet 1: 1049-1052, 1987) Mycobacterium bovis BCG (Fine and Rodrigues, Lancet 335: 1016-1020, 1990) und Vibrio cholerae (Levine and Kaper, Vaccine 11: 207-212, 1993) als Lebendvakzinen eingesetzt.

Beispielsweise wurden von Listeria monocytogenes, Salmonella enterica sv. Typhimurium und Typhi, sowie BCG derartige Varianten bereits als gut verträgliche Lebendimpfstoffe gegen Typhus und Tuberkulose eingesetzt. Diese Bakterien, einschließlich ihrer abgeschwächten Mutanten sind generell immunstimulierend und können eine gute zelluläre Immunantwort auslösen und wurden daher als Impfstoffträger eingesetzt.

Der Vorteil dieser Bakterien als Impfstoffträger ist, dass sie vor allem eine sogenannte Th1 Immunantwort induzieren (Hess and Kaufmann, FEMS Immunol Med Microbiol 23: 165-173, 1999). Diese Immunantwort zeichnet sich durch cytotoxische Lymphozyten (CTL) sowie durch die Anwesenheit spezifischer IFN-gamma sekretierender CD4+ T-Zellen (auch T-Helferzellen, Th) aus (Abbas et al., Nature 383: 787-793, 1996).

Beispielsweise stimuliert *L. monocytogenes* in besonderem Maße über die Aktivierung von TH1 Zellen die Proliferation von zytotoxischen T-Lymphozyten (CTL). Diese Bakterien liefern sezernierte Antigene direkt in das Cytosol Antigen-präsentierender Zellen (APC; Makrophagen und Dendritische Zellen), die ihrerseits die ko-stimulierenden Moleküle exprimieren und eine effiziente Stimulierung von T-Zellen auslösen. Die Listerien werden zum Teil in phagosomalen Kompartimenten abgebaut und die von diesen Trägerbakterien produzierten Antigene können daher einerseits über MHC Klasse II Moleküle präsentiert werden und damit zur Induktion von T-Helferzellen führen. Andererseits replizieren die Listerien nach Freisetzung aus dem Phagosom im Cytosol von APCs; von diesen Bakterien produzierte und sezernierte Antigene werden deshalb bevorzugt über den MHC Klasse I-Weg präsentiert, wodurch CTL Antworten gegen diese Antigene induziert werden. Außerdem konnte gezeigt werden, dass durch die Interaktion der Listerien mit Makrophagen, natürlichen Killerzellen (NK) und Neutrophilen Granulozyten die Expression solcher Cytokine (TNF-alpha, IFN-gamma, I1-2, IL-12; Unanue, Curr Opin Immunol, 9: 35-43, 1997; Mata and Paterson, J Immunol 163: 1449-14456,1999) induziert wird, für welche eine antitumorale Wirksamkeit nachgewiesen wurde.

Rekombinante Bakterien waren demzufolge in der Lage, gegen einen heterologen Tumor zu schützen (Medina et al., Eur J Immunol 29: 693-699, 1999; Pan et al., Cancer Res 59: 5264-5269, 1999; Woodlock et al., J Immunother 22: 251-259, 1999; Paglia et al., Blood 92: 3172-3176, 1998; Paglia et al., Eur J Immunol 27: 1570-1575, 1997; Pan et al., Nat Med 1: 471-477, 1995; Pan et al., Cancer Res 55: 4776-4779, 1995).

So konnte durch die Verabreichung von L. monocytogenes, welche transduziert waren zur Expression von Tumorantigenen, antigenspezifisch das Wachstum von experimentellen Tumoren gehemmt werden ( Pan et al., Nat Med 1: 471-477,1995; Cancer Res 59: 5264-5269,1999; Voest et al., Natl Cancer Inst 87: 581-586,1995; Beatty and Paterson, J Immunol 165: 5502-5508, 2000).

Virulenz-attenuierte *Salmonella enterica* Stämme, in welche Nukleotidsequenzen kodierend für Tumorantigene eingeführt worden waren, konnten als Tumorantigen-exprimierende bakterielle Träger nach oraler Verabreichung einen spezifischen Schutz gegen unterschiedliche experimentelle Tumoren bewirken (Medina et al., Eur J Immunol 30: 768-777, 2000; Zoller und Christ J Immunol 166: 3440-34450, 2001; Xiang et al., PNAS 97: 5492-5497, 2000).

Rekombinante *Salmonella* Stämme waren auch als prophylaktische Vakzine gegen Virusinfektionen (HPV; Benyacoub et al., Infect Immun 67: 3674-3679, 1999) und zur therapeutischen Behandlung eines durch ein Tumorvirus (HPV) immortalisierten Maustumors wirksam (Revaz et al., Virology 279: 354-360, 2001).

Für die Verwendung als Impfstoffträger wurden Verfahren entwickelt, Expressionsprodukte von in Bakterien eingeführten Nukleinsäuresequenzen auf der Zellmembran dieser Bakterien zu exprimieren oder von diesen Bakterien sekretieren zu lassen. Basis dieser Verfahren ist das Escherichia coli Hämolysinsystems HlyAs, welches den Prototyp eines Typ I Sekretionssystems von gramnegativen Bakterien darstellt. Mit Hilfe des HlyAs wurden Sekretionsvektoren entwickelt, die eine effiziente Ausschleusung von Proteinantigenen in Samonella enterica, Yersinia enterocolitica und Vibrio cholerae ermöglichen. Derartige Sekretionsvektoren enthalten die cDNA eines beliebigen Proteinantigens gekoppelt an die Nukleotidsequenz für das HlyA-Signalpeptid, für den Hämolysin-Sekretionsapparat, hlyB und hlyD und den hly-spezifischen Promoter. Mit Hilfe dieses Sekretionsvektors kann ein Protein beispielsweise auf der Oberfläche dieses Bakteriums exprimiert werden. Derartig genetisch modifizierte Bakterien induzieren als Vakzinen einen weitaus stärkeren Immunschutz als Bakterien, in welchen das von der eingeführten Nukleinsäure exprimierte Protein zellintern verbleibt (Donner et al EP 1015023 A; Gentschev et al., Gene 179: 133-140,1996; Vaccine 19: 2621-2618, 2001; Hess et al. PNAS 93: 1458-1463, 1996). Nachteil dieses Systems ist jedoch, dass durch Verwendung des Hly-spezifischen Promoters die Menge des von dem Bakterium exprimierten Proteins gering ist.

Weitere Transportsysteme in Bakterien stellen beispielsweise dar i) das Transportsignal für das S-layer Protein (Rsa A) von Caulobacter crescentus, bei welchem für die Sekretion und für die membranständige Expression - das C-terminale RsaA-Transportsignal zu verwenden ist (Umelo-Njaka et al., Vaccine 19: 1406-1415, 2001) und ii) das Transportsignal für das Internalin A von Listeria monocytogenes. Für die Sekretion ist das N-terminale Transportsignal und für die membranständige Expression das N-terminale Transportsignal mitsamt dem C-terminalen Teil enthaltend das für die Zellwandverankerung verantwortliche LPXTG-Motiv notwendig (Dhar et al., Biochemistry 39: 3725-3733, 2000).

Aus anderen Zusammenhängen ist das integrale Membranprotein TolC von E. coli bekannt. Dieses ist ein multifunktionelles, porenbildendes Protein der äußeren Membran von E. coli, das neben Funktionen wie z. B. der Aufnahme von Colicin E1 (Morona et al., J Bacteriol 153: 693-699, 1983) und der Sekretion von Colicin V (Fath et al., J Bacteriol 173: 7549-7556, 1991) auch als Rezeptor für den U3-Phagen dient (Austin et al., J Bacteriol 172: 5312-5325,1990). Dieses Protein findet sich nicht nur in E. coli, sondern in einer Vielzahl von Gram negativen Bakterien (Wiener, Structure Fold Des, 8: 171-5, 2000].

Die Kristallstruktur von dem TolC Protein zeigt, dass es als Homotrimer einen Tunnelkanal mit einer Länge von etwa 120 Angström bildet, wobei der größte Teil des Homodimers , die Tunneldomäne, im Periplasma lokalisiert ist und nur zwei kleine Schleifen (Aminosäuren 52-61 und 257-279) auf der Oberfläche des Bakteriums angesiedelt sind (Koronakis et al., Nature 405: 914-919, 2000). Das *tolC*-Gen besitzt die Nukleotidsequenz, publiziert von Niki et al, Nucleotide sequence of the tolC gene of Escherichia coli, Nucleic Acids Res. 18 (18), 5547 (1990). TolC ist Teil von zumindest vier unterschiedlichen bakteriellen Exportsystemen, indem es den Membrantunnel darstellt, durch welchen der Export des bakteriellen Proteins ermöglicht wird. Beispielsweise erlaubt im HlyA Transportsystem die Verbindung zwischen HlyD und dem periplasmatischen Ende des TolC den Export des Hämolysins vom HlyD in den Membrantunnel des TolC (Gentschev et al Trends in Microbiology 10: 39-45, 2002).

### Technisches Problem der Erfindung.

Der Erfindung liegt das technische Problem zu Grunde, ein Transportsystem anzugeben, mit dessen Hilfe ein Expressionsprodukt mit höherer Effizienz auf einer äußeren Zellmembran eines Bakteriums präsentiert werden kann.

Grundkonzeption der Erfindung und bevorzugte Ausführungsformen.

Zur Lösung dieses technischen Problems lehrt die Erfindung eine Nukleotidsequenz codierend für ein TolC sowie eine definierte Aminosäurensequenz, wobei die definierte Aminosäurensequenz in den permissiven, membranaußenseitigen Bereich des TolC insertiert ist.

Mit der Erfindung ist ein neues Transportsystem in gram-negativen Bakterien geschaffen, mit Hilfe dessen größere Mengen eines von einem Gen innerhalb des Bakteriums exprimierten Proteins auf die äußere Zellmembran des Bakteriums transportiert werden können, als es mit den bisher bekannten Transportsystemen möglich war. Überraschenderweise erlaubt das Transportsystem für das TolC-Protein von Escherichia coli eine weitaus stärkere membranständige Expression eines (beliebigen) Peptids oder Proteins, als es von den bisherigen Transportproteinen bekannt war, und 5 zwar bei Vielzahl Gram negativer Bakterien. Die membranständige Expression der definierten Aminosäurensequenz bzw. Genproduktes wird dabei allein durch das TolC erreicht.

Eine definierte Aminosäuresequenz kann sein ein beliebiges vorgegebenes Peptid oder ein Protein, ein beliebiger pharmazeutischer Wirkstoff, ein beliebiges Antigen, ein beliebiger Antikörper, oder ein beliebiger Ligand.

Das TolC kann ein (wildtypisches) TolC Protein gemäß ACCESSION X54049, worauf hiermit ausdrücklich Bezug genommen wird, oder eine (vorzugsweise N-terminale) Teilsequenz hiervon oder eine Mutante des Proteins oder der Teilsequenz sein, wobei für die Teilsequenz oder die Mutante die Transportfunktionalität erhalten ist. N-terminale Teilsequenz meint hierbei eine Teilsequenz beginnend im N-terminalen Bereich Aminosäuren 1 bis 50 des TolC Proteins und endend am C-terminalen Ende einer Schleife, welche auf der Oberfläche des Bakteriums angesiedelt ist. Bevorzugt sind somit das N-terminale Transportsignal von TolC, aber auch der mittlere Teil des Proteins, welcher die extrazellulären Bereiche von TolC darstellt. Eine Mutante kann eine Insertion, Deletion oder Substitution umfassen, solange die Transportfunktionalität hiervon nicht deutlich reduziert wird.

Für bestimmte Anwendungsfälle kann es sich empfehlen, wenn die definierte Aminosäurensequenz einseitig oder beidseitig über eine Spacersequenz insertiert ist. Dies wird allerdings nur dann hilfreich sein, wenn die definierte Aminosäurensequenz in einer bestimmten räumlichen Struktur präsentiert werden soll, beispielsweise im Falle eines Antigens, dies jedoch nicht durch die definierte Aminosäurensequenz selbst aus sterischen oder konfigurativen Gründen in gewünschtem Maße erfolgt. Dann kann eine Spacersequenz insbesondere durch eine natürlicherweise an die definierte Aminosäurensequenz anschließende Sequenz gebildet sein, wodurch die definierte Aminosäurensequenz so gefaltet wird, wie in dem natürlichen Antigen. Die Spacersequenz kann aber auch künstlich sein, sofern dadurch eine gewünschte Präsentation und/oder Faltung der definierten Aminosäurensequenz erreicht wird. Dies läßt sich mittels theoretischer Verfahren unschwer berechnen unter Berücksichtigung der räumlichen Bedingungen an der Stelle der Insertion in dem TolC.

Im Einzelnen ist es bevorzugt, wenn die definierte Aminosäurensequenz im N-terminalen Bereich des TolC, insbesondere im Bereich der Aminosäuren 52 bis 61, und/oder 257 bis 279 (jeweils bezogen auf das TolC Protein), insertiert ist.

Gegenstand der Erfindung ist weiterhin ein Plasmid enthaltend eine erfindungsgemäße Nukleotidsequenz sowie ein Protein oder Peptid codiert durch eine erfindungsgemäße Nukleotidsequenz.

Die Erfindung lehrt weiterhin ein Bakterium enthaltend eine erfindungsgemäße Nukleotidsequenz, wobei das TolC den Transport der definierten Aminosäuresequenz auf die Membran des Bakteriums bewirkt. Mit anderen Worten ausgedrückt, in dem Bakterium wird die membranständige Expression eines Genproduktes durch das TolC Protein bewirkt. Gegenstand der Erfindung ist somit auch ein gramnegatives Bakterium, welches mindestens eine Nukleotidsequenz enthält, welche für mindestens eine definierte Aminosäuresequenz und für mindestens ein *E*. *coli* TolC-Genprodukt kodiert. Dieses *E*.*coli* TolC-Genprodukt ist bevorzugterweise wildtypisch. Gegenstand der Erfindung sind jedoch auch mutierte E.coli TolC Genprodukte, bei denen die Transportsignalakivität erhalten geblieben ist. Bevorzugterweise ist das Bakterium ausgewählt aus der Gruppe bestehend aus "Salmonella spp, Escherichia coli, Vibrio cholerae, Pseudomonas aeruginosa, Shigella spp. und Yersinia spp.".

Erfindungsgemäße Nukleotidsequenzen bzw. Bakterien sind für verschiedenste Anwendungen einsetzbar. So lehrt die Erfindung auch eine pharmazeutische Zusammensetzung enthaltend ein erfindungsgemäßes Bakterium sowie, optional, zumindest einen physiologisch verträglichen Trägerstoff, wobei die definierte Aminosäuresequenz nach Maßgabe einer vorgegebenen, in einem Organismus zu bindenden Substanz ausgewählt ist. Mittels einer solchen pharmazeutischen Zusammensetzung lassen sich den normalen zellulären Stoffwechsel störende Substanzen, beispielsweise exogene Giftstoffe oder mutationsbedingte endogene Substanzen, wie Onkogene, binden und so hemmen. Auch lassen sich durch Bindung von bestimmten zellulären Zielsubstanzen Stoffwechselprozesse modulieren durch Entzug von normalen oder krankheitsbedingt hochregulierten Komplexpartnern. Dadurch wird beispielsweise eine definierte Assoziation gehemmt und das damit verbundene Shuttle herunterreguliert. Ein solcher Prozeß kann wiederum zur hochregulation von anderen verknüpften Prozessen genutzt werden. Insofern braucht die definierte Aminosäurensequenz lediglich nach Maßgabe des mit hoher Spezifität zu inhibierenden Zielmoleküls ausgewählt werden. Eine solche pharmazeutische Zusammensetzung dient somit letztendlich therapeutischen Zwecken.

Eine zu Impfzwecken geeignete pharmazeutische Zusammensetzung enthält ein erfindungsgemäßes Bakterium sowie, optional, zumindest einen physiologisch verträglichen Trägerstoff, wobei die definierte Aminosäuresequenz eine Immunisierungssequenz ist. Eine Immunisierungssequenz stimuliert in einem Organismus die Bildung von Antikörpern gegen ein natürliches Antigen, welches als Teilsequenz die Immunisierungssequenz enthält oder daraus besteht.

Für diagnostische Zwecke lehrt die Erfindung ein diagnostisches Kit enthaltend ein Bakterium nach einem der Ansprüche 7 bis 9, wobei die definierte Aminosäuresequenz eine zu bestimmende Markersubstanz spezifisch bindet. Wird beispielsweise einem Organismus eine Gewebe- oder Fluidprobe entnommen und wird diese Probe, ggf. nach einer Vorbehandlung mit Abtrennung unerwünschter Probenbestandteile, mit dem Bakterium inkubiert, so lassen sich Bindungsereignisse an der definierten Aminosäuresequenz detektieren und im Falle eines Bindungsereignis ist festgestellt, daß die an die definierte Aminosäurensequenz spezifisch bindende Substanz in der Probe enthalten ist. Der Nachweis von Bindungsereignissen kann dabei auf verschidenste, dem Druchschnittsfachmann vertraute Weise erfolgen.

Schließlich lehrt die Erfindung ein präparatives Bindungsmittel enthaltend ein erfindungsgemäßes Bakterium, wobei die definierte Aminosäuresequenz eine aus einer Lösung abzutrennende Zielsubstanz spezifisch bindet. Mit einem solchen Bindungsmittel lassen sich aus einer Vorlage einerseits unerwünschte Substanzen dadurch spezifisch entfernen, daß die Lösung mit dem Bakterium inkubiert wird und das Bakterium nach Abtrennung verworfen wird. Andererseits kann in entsprechender Weise eine Abtrennung bzw. Anreicherung einer Zielsubstanz erfolgen, nämlich indem nach der Inkubation die Zielsubstanz von dem Bakterium eluiert wird. Auch in diesen Zusammenhängen kann die Erfindung zur Abtrennung und/oder Anreicherung von Antigenen, von Antikörpern, Peptiden, Proteinen oder Liganden eingesetzt werden.

### Ausführungsbeispiele

### Beispiel 1: Herstellung des TolC Vektors

Das *tolC*-Gen aus *E*. *coli* wurde inklusive seines wildtypischen Promotors mittels PCR (1 min 94° C, 1 min 66° C, 1 min 30 s 72° C) mit den Oligonukleotiden 5'TolC (5'-TAACGCCCTATGTCGACTAACGCCAACCTT-3') und 3'TolC (5'- AGAG-GATGTCGACTCGAAATTGAAGCGAGA-3') aus dem Plasmid pAX629 (C. Wandersman, Institute Pasteur, Paris) amplifiziert. Dabei wurde an beiden Enden eine zusätzliche SalI-Schnittstelle eingeführt. Das gereinigte PCR-Produkt (QIAquick PCR Purification Kit - Qiagen, Hilden, Germany) wurde mit der Restriktionsendonuklease *Sal*I verdaut und in den mit *Sal*Ivorgespaltenen Vektor pBR322 kloniert. Der so konstruierte Vektor wurde als pBR322*tol*C bezeichnet. Die Funktionalität des klonierten *tolC*-Gens wurde anschließend in mehreren Tests überprüft.

### Beispiel 2: Einfügung einer Antigensequenz in die Sequenz des TolC Proteins

In der *tol*C-Sequenz, die für eine der extrazellulären Schleifen kodiert, wurde eine *Kpn*I-Schnittstelle identifiziert. Diese wurde für die Klonierung von antigenen Peptidsequenzen des p60-Proteins (*iap*-Gen) von *Listeria monocytogenes* verwendet und erlaubte eine Insertion von Fremdantigenen nach Aminosäure 271 des reifen TolC-Proteins.

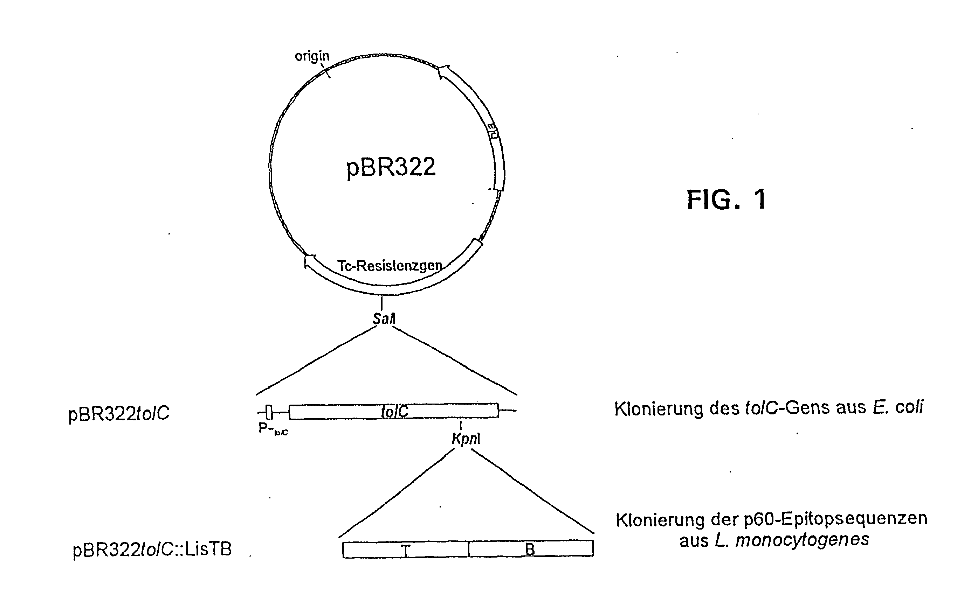

Die *iap*-Sequenz, die für ein B-Zellepitop (Aminosäuren 291-301) und ein CD4-restringiertes T-Zellepitop (Aminosäuren 301-312) des p60-Proteins kodiert, wurde als ein *Kpn*I- Fragment in den mit *Kpn*I-vorgeschnittenen Vektor pBR322*tolC* kloniert (Abb. 1). Das so entstandene Plasmid wurde als pBR322*tolC*::LisTB bezeichnet.

Die Figur 1 zeigt die Klonierungsstrategie für die Insertion der p60-spezifischen Epitopsequenzen in das wildtypische, plasmidkodierte *E. coli tolC*-Gen auf dem Vektor pBR322. Es sind: *bla -* Ampicillinresistenzgen; Tc - Tetracyclin; T - *L. monocytogenes* p60-T-Zellepitop (AS 301-312); B - *L. monocytogenes* p60-B-Zellepitop (AS 291-301); P*tolC -* wildtypischer *E*. *coli tolC*-Promotor.

### Beispiel 3: Expression des Antigens auf der Membran eines Gram-negativen Bakteriums (Escherichia coli)

Die Expression der Epitope des p60-Proteins aus *L*. *monocytogenes* innerhalb des TolC-Proteins wurde in einem Western Blot nachgewiesen. Hierzu wurden Zell- Lysat-Proteine von *E*. *coli* CC118*tolC*, *E*. *coli* CC118*tolC*/pBR322*tolC* und von *E*. *coli* CC118*tolC* /pBR322*tolC*::LisTB in der späten logarithmischen Phase isoliert. Die aufgetragenen Gesamtzellproteine entsprachen etwa 100 Mio Bakterien. Die Proteine wurden in einem 15%igen SDS-Polyacrylamidgel aufgetrennt und die Expression der chimären TolC-Proteine bzw. der inserierten Epitope zum einen mit einem polyklonalen Serum gegen das TolC-Protein und zum anderen mit dem monoklonalen Antikörper K317 (Rowan et al., J Clin Microbiol 38: 2643-2648, 2000), der spezifisch gegen das B-Zell-Epitop aus *L*. *monocytogenes* (Abb. 2B.) gerichtet ist, detektiert.

Erwartungsgemäß konnte im Zellysat von *E*. *coli* CC118*tolC* kein TolC-Protein nachgewiesen werden, was auf eine Mutation im chromosomalen *tolC*-Gen in diesem Stamm zurückzuführen ist (Schlor et al., Mol Gen Genet 256: 306-319, 1997). Die Komplementation mit pBR322*tolC* führte in diesem Stamm zur Expression des 52 kDa großen TolC-Proteins. Die Insertion der *L*. *monocytogenes*-Epitope in das TolC-Protein beeinflusste die Expression von TolC nicht und führte zu einer leichten Größenverschiebung des chimären Proteins von etwa 3 kDa.
Die Expression der p60-spezifischen Epitope in *E*. *coli* CC118*tolC*/pBR322*tolC*::LisTB konnte mit dem monoklonalen p60-Antikörper K317 bestätigt werden.

### Beispiel 4: Nachweis der exponierten Lokalisation der L. monocytogenes p60-Epitope in Salmonella enteritidis SM6T (tolC)

Da sich die Insertionsstelle der beiden listeriellen p60-Epitope in einer extrazellulären Schleife von TolC nach Aminosäure 271 des reifen Proteins befand, sollten diese an der Oberfläche von *S*. *enteritidis* SM6T (Stone et al., Mol Microbiol 17: 701-712, 1995) exponiert vorliegen. Die definitive extrazelluläre Lokalisation der p60-spezifischen Epitope in *S. enteritidis* SM6T wurde durch indirekte Immunfluoreszenz überprüft.
Je 25 µl einer Übernachtkultur von *S. enteritidis* SM6T/pBR322*tolC* und *S*. *enteritidis* SM6T/ pBR322*tolC*::LisTB wurden auf Objektträger aufgetropft und luftgetrocknet. Die Zellen wurden mit dem monoklonalen p60-Antikörper K317 (1:200) gefärbt und gebundene Antikörper anschließend mit einem FITC-markierten sekundären anti-Maus-Serum (Dianova, Germany, Arbeitstiter: 1:40) detektiert.

Die fluoreszenzmikroskopische Analyse bestätigte die extrazelluläre Lokalisation der *L. monocytogenes*-spezifischen Epitope im Stamm *S*. *enteritidis*
SM6T/pBR322*tolC*::LisTB.

### Beispiel 5: Immunisierungsversuche mit dem gramnegativen Bakterium und Analyse der protektiven Immunantworten nach Infektion mit wildtypischen L. monocytogenes

Um zu untersuchen, ob die exponierte Expression des T-Zellepitops aus dem p60-Protein von *L. monocytogenes* im murinen Listeriosemodell zu einem Schutz führt, wurden 8 weibliche Balb/c-Mäuse (Charles River, Sulzfeld, Germany) im Alter von sechs Wochen mit einer Dosis von 1x107 *S. enteritidis*/ pBR322*tolC*::LisTB oral immunisiert. Zur Kontrolle wurden 5 weibliche Mäuse mit *S*. *enteritidis* SM6T oral immunisiert. Die Tiere wurden drei Wochen später ein zweites Mal mit derselben Bakteriendosis immunisiert.

Der Immunisierungserfolg wurde fünf Wochen nach der ersten Immunisierung in einem Immunoblot überprüft, auf dem Überstandsproteine von *Listeria monocytogenes* aufgetragen waren. Dabei konnten anti-p60-spezifische Antikörper im Serum der mit *S*. *enteritidis* /pBR322*tolC*::LisTB immunisierten Mäuse nachgewiesen werden.

Drei Wochen nach der zweiten Immunisierung wurden die Tiere mit 5x104 *L*. *monocytogenes* EGD, der 5-fachen LD50, intravenös infiziert. Während die Überlebensrate bei den mit *S*. *enteritidis*/pBR322*tolC*::LisTB immunisierten Balb/c-Mäuse nach intravenöser Infektion mit *L*. *monocytogenes* EGD 88% betrug, lag in der Kontrollgruppe die Überlebensrate bei nur 20 %.

Damit führte die Expression der p60-spezifischen Epitope innerhalb eines extrazellulären Loops von TolC im attenuierten *S*. *enteritidis*-Trägerstamm SM6T zur Induktion von *Listeria monocytogenes*-spezifischen Immunantworten, die in der Lage waren, Balb/c-Mäuse gegen eine in der Regel tödliche Infektion zu schützen. Da die Induktion von Antikörpern gegen das B-Zellepitop aus dem p60-Protein in einem Western Blot nachgewiesen werden konnte, ist es naheliegend, dass eine Immunreaktion unter Beteiligung der Antikörper den hier beobachteten Schutz der Mäuse vor einer ansonsten letalen Infektion mit *L. monocytogenes* bewirkt hat.

## Patentansprüche

1. Nukleotidsequenz kodierend für ein TolC sowie eine definierte Aminosäurensequenz, wobei die definierte Aminosäurensequenz im Bereich der extrazellulären Schleifen des TolC insertiert ist und ausgewählt ist aus der Gruppe bestehend aus einem beliebigen vorgegebenen Peptid oder Protein, einem beliebigen pharmazeutischen Wirkstoff, einem beliebigen Antigen, einem beliebigen Antikörper oder einem beliebigen Liganden, sowie einer Immunisierungssequenz.

2. Nukleotidsequenz gemäß Anspruch 1, wobei das TolC TolC-Protein gemäß ACCESSION X54049 oder eine vorzugsweise N-terminale Teilsequenz hiervon oder eine Mutante des Proteins oder der Teilsequenz ist und wobei für die N-terminale Teilsequenz oder die Mutante die Transportfunktionalität erhalten ist.

3. Nukleotidsequenz gemäß einem der Ansprüche 1 bis 2, wobei die definierte Aminosäurensequenz einseitig oder beidseitig über eine Spacersequenz insertiert ist.

4. Nukleotidsequenz gemäß einem der Ansprüche 1 bis 3, wobei die definierte Aminosäurensequenz im N-terminalen Bereich des TolC, insbesondere im Bereich der Aminosäuren 52 bis 61, und/oder 257 bis 279 (jeweils bezogen auf das TolC-Protein), insertiert ist.

5. Plasmid enthaltend eine Nukleotidsequenz gemäß einem der Ansprüche 1 bis 4.

6. Protein oder Peptid kodiert durch eine Nukleotidsequenz gemäß einem der Ansprüche 1 bis 5.

7. Bakterium enthaltend eine Nukleotidsequenz gemäß einem der Ansprüche 1 bis 5, wobei das TolC den Transport der definierten Aminosäurensequenz auf die Membran des Bakteriums bewirkt.

8. Bakterium gemäß Anspruch 7, wobei die definierte Aminosäurensequenz ein Peptid, ein Protein, einen pharmazeutischen Wirkstoff, ein Antigen, einen Antikörper oder einen Liganden darstellt.

9. Bakterium gemäß einem der Ansprüche 7 bis 8, ausgewählt aus der Gruppe bestehend aus "gram-negatives Bakterium, Salmonella spp., Escherichia coli, Vibrio cholerae, Pseudomonas aeruginosa, Shigella spp. und Yersinia spp.".

10. Pharmazeutische Zusammensetzung enthaltend ein Bakterium gemäß einem der Ansprüche 7 bis 9 sowie, optional, zumindest einen physiologisch verträglichen Trägerstoff, wobei die definierte Aminosäurensequenz nach Maßgabe einer vorgegebenen, in einem Organismus zu bindenden Substanz ausgewählt ist.

11. Pharmazeutische Zusammensetzung enthaltend ein Bakterium gemäß einem der Ansprüche 7 bis 9 sowie, optional, zumindest einen physiologisch verträglichen Trägerstoff, wobei die definierte Aminosäurensequenz eine Immunisierungssequenz ist.

12. Diagnostisches Kit enthaltend ein Bakterium gemäß einem der Ansprüche 7 bis 9, wobei die definierte Aminosäurensequenz eine zu bestimmende Markersubstanz spezifisch bindet.

13. Präparatives Bindungsmittel enthaltend ein Bakterium gemäß einem der Ansprüche 7 bis 9, wobei die definierte Aminosäurensequenz eine aus einer Lösung abzutrennende Zielsubstanz spezifisch bindet.

## Claims

1. Nucleotide sequence coding for a TolC and a defined amino acid sequence, where the defined amino acid sequence is inserted in the region of the extracellular loop of the TolC and is selected from the group consisting of any desired predetermined peptide or protein, any desired active pharmaceutical ingredient, any desired antigen, any desired antibody or any desired ligand, and an immunizing sequence.

2. Nucleotide sequence according to Claim 1, where the TolC is TolC protein according to ACCESSION X54049 or a preferably N-terminal partial sequence thereof or a mutant of the protein or of the partial sequence, and where the transport functionality is retained for the N-terminal partial sequence or the mutant.

3. Nucleotide sequence according to either of Claims 1 and 2, where the defined amino acid sequence is inserted via a spacer sequence at one or both ends.

4. Nucleotide sequence according to any of Claims 1 to 3, where the defined amino acid sequence is inserted in the N-terminal region of the TolC, in particular in the region of amino acids 52 to 61, and/or 257 to 279 (in each case based on the TolC protein).

5. Plasmid comprising a nucleotide sequence according to any of Claims 1 to 4.

6. Protein or peptide encoded by a nucleotide sequence according to any of Claims 1 to 5.

7. Bacterium comprising a nucleotide sequence according to any of Claims 1 to 5, where the TolC brings about transport of the defined amino acid sequence to the membrane of the bacterium.

8. Bacterium according to Claim 7, where the defined amino acid sequence represents a peptide, a protein, an active pharmaceutical ingredient, an antigen, an antibody or a ligand.

9. Bacterium according to either of Claims 7 to 8, selected from the group consisting of "gram-negative bacterium, Salmonella spp., Escherichia coli, Vibrio cholerae, Pseudomonas aeruginosa, Shigella spp. and Yersinia spp.".

10. Pharmaceutical composition comprising a bacterium according to any of Claims 7 to 9 and, optionally, at least one physiologically tolerated carrier, where the defined amino acid sequence is selected according to a predetermined substance to be bound in an organism.

11. Pharmaceutical composition comprising a bacterium according to any of Claims 7 to 9 and, optionally, at least one physiologically tolerated carrier, where the defined amino acid sequence is an immunizing sequence.

12. Diagnostic kit comprising a bacterium according to any of Claims 7 to 9, where the defined amino acid sequence specifically binds a marker substance to be determined.

13. Preparative binding composition comprising a bacterium according to any of Claims 7 to 9, where the defined amino acid sequence specifically binds a target substance to be removed from a solution.

## Revendications

1. Séquences de nucléotides codant pour une TolC ainsi qu'une séquence d'acides aminés définie, dans laquelle la séquence d'acides aminés définie est insérée dans la zone de la boucle extracellulaire de la TolC et choisie dans le groupe constitué par un peptide ou une protéine quelconque donné(e), un principe actif pharmaceutique quelconque, un antigène quelconque, un anticorps quelconque ou un ligand quelconque ainsi qu'une séquence d'immunisation.

2. Séquence de nucléotides selon la revendication 1,
**caractérisée en ce que**
la TolC est une protéine TolC selon le numéro d'accès X54049, ou une séquence partielle de préférence N-terminale de celle-ci, ou un mutant de la protéine ou de la séquence partielle, et qui contient la fonction de transport pour la séquence partielle N-terminale ou le mutant.

3. Séquence de nucléotides selon la revendication 1 ou 2,
**caractérisée en ce que**
la séquence d'acides aminés définie est insérée de façon unilatérale ou bilatérale sur une séquence intercalaire.

4. Séquence de nucléotides selon l'une des revendications 1 à 3,
**caractérisée en ce que**
la séquence d'acides aminés définie est insérée dans la zone N-terminale de la TolC, notamment dans le domaine des acides aminés 52 à 61, et/ou 257 à 279 (chaque fois rapportés à la protéine TolC).

5. Plasmide contenant une séquence de nucléotides selon l'une des revendications 1 à 4.

6. Protéine ou peptide codé(e) par une séquence de nucléotides selon l'une des revendications 1 à 5.

7. Bactérie contenant une séquence de nucléotides selon l'une des revendications 1 à 5,
**caractérisée en ce que**
la TolC effectue le transport de la séquence d'acides aminés définie sur la membrane de la bactérie.

8. Bactérie selon la revendication 7,
**caractérisée en ce que**
la séquence d'acides aminés définie représente un peptide, une protéine, un principe actif pharmaceutique, un antigène, un anticorps ou un ligand.

9. Bactérie selon l'une des revendications 7 à 8, choisie dans le groupe constitué par une bactérie à Gram négatif, Salmonella spp., Escherichia coli, le vibrion cholérique, Pseudomonas aeruginosa, Shigella spp. et Yersinia spp.

10. Composition pharmaceutique contenant une bactérie selon l'une des revendications 7 à 9 ainsi que, de manière optimale, au moins un excipient physiologiquement acceptable, dans laquelle la séquence d'acides aminés définie est choisie conformément à une substance donnée destinée à se fixer dans un organisme.

11. Composition pharmaceutique contenant une bactérie selon l'une des revendications 7 à 9 ainsi que, de manière optimale, au moins un excipient physiologiquement acceptable, dans laquelle la séquence d'acides aminés définie est une séquence d'immunisation.

12. Trousse de diagnostic contenant une bactérie selon l'une des revendications 7 à 9,
**caractérisée en ce que**
la séquence d'acides aminés définie se fixe spécifiquement à une substance de marquage déterminée.

13. Liant de préparation contenant une bactérie selon l'une des revendications 7 à 9,
**caractérisé en ce que**
la séquence d'acides aminés définie se fixe spécifiquement à une substance cible à séparer d'une solution.
